# EUROPEAN PATENT SPECIFICATION

(11) **EP 2 620 447 B1**
(45) Date of publication and mention of the grant of the patent: **09.09.2015**
(21) Application number: 13159236.2
(22) Date of filing: 04.02.2009
(51) Int. Cl.: C07K 14/47

(54) **Vector(s) containing an inducible gene encoding a CDK4/CDK6 inhibitor useful for treating neurodegenerative disorders**
Vektor(en) mit einem CDK4/CDK6-Inhibitor kodierenden induzierbaren Gen für die Behandlung von neurodegenerativen Erkrankungen
Vecteur(s) contenant en gène inductible codant un inhibiteur CDK4/CDK6 utile pour traiter les troubles neuro-dégenératifs

(43) Date of publication of application: 31.07.2013
(62) Divisional of application: 09001521.5
(73) Proprietor: Universität Leipzig, 04109 Leipzig (DE)
(72) Inventor: Arendt, Thomas, 04109 Leipzig (DE); Ueberham, Uwe, 04289 Leipzig (DE)
(74) Representative: Schüssler, Andrea

(56) References cited:
- DIRKS P B ET AL: "RETINOIC ACID AND THE CYCLIN DEPENDENT KINASE INHIBITORS SYNERGISTICALLY ALTER PROLIFERATION AND MORPHOLOGY OF U343 ASTROCYTOMA CELLS", ONCOGENE, NATURE PUBLISHING GROUP, GB, vol. 15, no. 17, 1 January 1997 (1997-01-01), pages 2037-2048, XP008049161, ISSN: 0950-9232, DOI: 10.1038/SJ.ONC.1201392
- UEBERHAM U ET AL: "Expression of p16INK4a protein protects differentiated cells from apoptosis", ABSTRACTS OF THE ANNUAL MEETING OF THE SOCIETY FOR NEUROSCIENCE, SOCIETY FOR NEUROSCIENCE, WASHINGTON, DC, US, vol. 27, no. 2, 1 January 2001 (2001-01-01), page 2318, XP009117951, ISSN: 0190-5295
- UEBERHAM UWE ET AL: "Inducible neuronal expression of transgenic TGF-beta 1 in vivo: dissection of short-term and long-term effects", EUROPEAN JOURNAL OF NEUROSCIENCE, OXFORD UNIVERSITY PRESS, GB, vol. 22, no. 1, 1 July 2005 (2005-07-01), pages 50-64, XP002531008, ISSN: 0953-816X, DOI: 10.1111/J.1460-9568.2005.04189.X
- PARK DAVID S ET AL: "Cyclin dependent kinase inhibitors and dominant negative cyclin dependent kinase 4 and 6 promote survival of NGF-deprived sympathetic neurons", JOURNAL OF NEUROSCIENCE, NEW YORK, NY, US, vol. 17, no. 23, 1 December 1997 (1997-12-01), pages 8975-8983, XP002531003, ISSN: 0270-6474
- DUBENSKY T W ET AL: "SINDBIS VIRUS DNA-BASED EXPRESSION VECTORS: UTILITY FOR IN VITRO AND IN VIVO GENE TRANSFER", JOURNAL OF VIROLOGY, THE AMERICAN SOCIETY FOR MICROBIOLOGY, US, vol. 70, no. 1, 1 January 1996 (1996-01-01), pages 508-519, XP002039561, ISSN: 0022-538X
- CHEN XI ET AL: "Senescence-like changes induced by expression of p21Waf1/Cip1 in NIH3T3 cell line", CELL RESEARCH - XIBAO YANJIU, NATURE PUBLISHING GROUP, GB, CN, vol. 12, no. 3-4, 1 September 2002 (2002-09-01), pages 229-233, XP002531000, ISSN: 1001-0602, DOI: 10.1038/SJ.CR.7290129
- ARENDT T ET AL: "Neuronal expression of cycline dependent kinase inhibitors of the INK4 family in Alzheimer's disease", JOURNAL OF NEURAL TRANSMISSION, SPRINGER VERLAG, VIENNA, AT, vol. 105, 1 January 1998 (1998-01-01), pages 949-960, XP002431095, ISSN: 0300-9564, DOI: 10.1007/S007020050104

## Description

The present invention provides (a) vector(s) containing (a) a gene encoding (i) a CDK4/CDK6 inhibitor of the INK4 family and (b) a gene encoding a transactivator protein for said promoter useful for treating neurodegenerative disorders. This vector can be transferred into cells where it will exert its protective function to (i) prevent cell death or to (ii) slow down progression of cell death. This vector construct can be used in therapeutic applications to prevent neurodegenerative disorders or to slow down their progression with therapeutic efficacy. Thus, in a preferred embodiment, the present invention is based on a gene therapeutic approach that affects cell cycle regulation of targeted cells. Moreover, existing risks with gene therapy and specific challenges for gene therapy posed by the central nervous system will be further met through (i) viral or (ii) non-viral vectors for safe gene transfer, (iii) cell type specific recognition systems, (iv) cell-type specific expression system and (v) controlled delivery by convection-enhanced delivery, preferably, a combination of (i) to (v).

Alzheimer's disease (AD) is an age-associated incurable neurodegenerative disorder of higher age which puts an enormous socio-economic burden on aging society. Within less than 20 years (2025) about one-third of the population in the EU will be older than 65 years and about one quarter will be over 80 years and, thus, be at risk for dementing disorders. The number of demented patients in Europe, currently estimated at about 8 million, will rise to 14 millions, by 2050.

Worldwide, demented patients will increase from currently 24.3 to 42 millions by 2020 with doubling every 20 years to 81 millions by 2040. Rates of increase in developed countries are forecast by 100% between 2001 and 2040, but by more than 300% in India, China and their Asian neighbours (FERRI ET AL., 2005). Annual costs of over € 160 billion in the EU already today make AD the third most expensive disease in the world. Medicare costs for AD will rise to € 240 billion in the EU by 2025. The lifetime risk for AD between 65 and 100 years is 33% for men and 45% for women (VAN DER FLIER AND SCHELTENS, 2005).

AD is characterized by a progressive neurodegeneration, a process of progressive structural desintegration that eventually results in neuronal death. This loss of neurons is associated with typical, albeit not specific neuropathological hallmarks, i.e. the deposition of abnormal molecular aggregates in form of intracellular neurofibrillar tangles and extracellular neuritic plaques. Neurofibrillar tangles are made up by 'paired helical filaments' composed by the microtubule-associated protein tau in a hyperphosphorylated form. Neuritic plaques consist of aggregated Aß-peptide that derives by proteolytic cleavage from the much larger Amyloid Precursor Protein (APP). The pathogenetic role of both neurofibrillary tangles and neuritic plaques still remains unclear. As so far, however, evidence for a causative role of these molecular deposits in the process of cell death is lacking, it is likely that they represent the result rather than the cause of neurodegeneration. Therefore, their suitability as molecular targets for prevention and/or treatment of AD might be very limited.

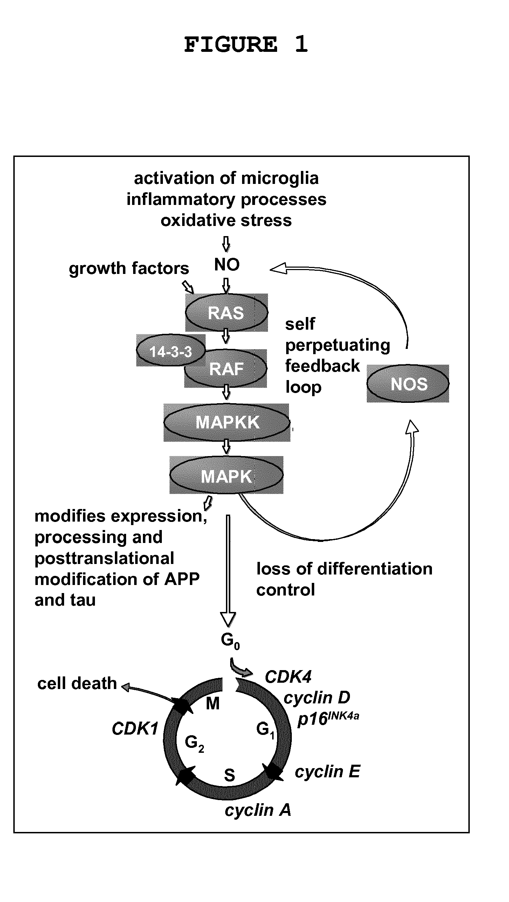

Neurodegeneration in AD is associated with aberrant structural neuronal plasticity, characterised by neuronal sprouting and re-organisation of cytokeletal proteins (ARENDT ET AL., 1995A, B, c; 1997). These processes are intracellularly mediated through abnormal activation of the Ras-MAP-kinase-pathway (Figure 1). This pathway is activated at very early stages of the disease and prior to any neurofibrillary pathology or accumulation of Aß (GÄRTNER ET AL., 1999). Distribution and progression of neurodegeneration throughout different brain areas in the course of AD, moreover, matches the pattern of neuronal plasticity (i.e. brain areas with a high degree of plasticity are most early involved while areas with a low degree of plasticity are only affected at most advanced stages), (ARENDT ET AL., 1998), indicating a link between neurodegeneration and molecular mechanisms involved in mediating structural plasticity.

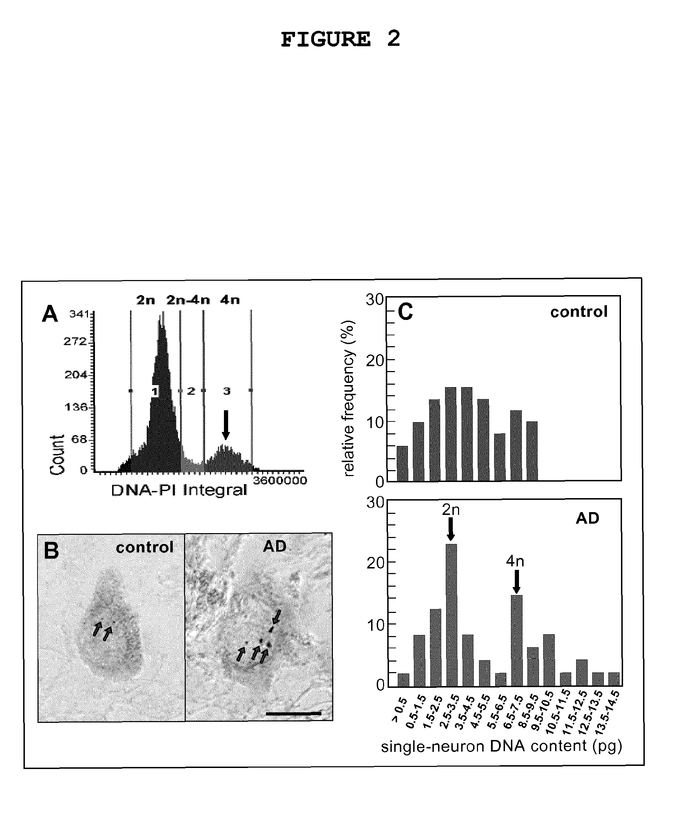

The aberrant activation of the Ras-MAP-kinase-pathway, being a mitogentic signalling mechanism involved in mediating structural plasticity (HEUMANN ET AL., 2000; ARENDT ET AL., 2004), apparently triggers a variety of down-stream effects not compatible with the terminally differentiated stage of a neuron in the mature brain, including reactivation of the cell-cycle. As indicated by re-expression of cell-cycle phase specific marker proteins (ARENDT ET AL., 1996; ARENDT 2000, 2001, 2003), neurons leave the Go-phase and progress until the S-phase and beyond. With three independent methods clear evidence for DNA replication in neurons during the process of neurodegeneration in AD could be obtained (Figure 2, MOSCH ET AL., 2007). DNA replication does not occur in areas spared by neurodegeneration. As there are no indications for progression into M-phase and beyond, very likely neurons die at the G₂-M transition (Figure 1).

Currently, distinct causes of the disease are still unknown, and there is neither an effective prevention of risk factors nor a treatment of the disorder. AD is one of the leading causes of disability, and represents the fastest growing area of unmet medical need. Finding a treatment that could delay the onset of AD by five years would cut the number of individuals with AD by half after 50 years. Preventing or even only slowing down progression of neurodegeneration will, thus, considerably improve the quality of life of the aging population and optimize the medical service utilization and the cost effectiveness of care.

Thus, the technical problem underlying the present invention is to provide means suitable for treating or preventing neurodegenerative disorders like AD.

The solution of said technical problem is achieved by providing the embodiments characterized in the claims. Neuronal cell death both during brain development and neurodegeneration is accompanied by re-activation of the cell cycle as evidenced by re-expression of cell-cycle regulators and partial or even complete replication of DNA. The observation that this process of cell cycle re-activation is related to cell death under such diverse conditions such as developmental cell death and neurodegeneration of various origins such as AD, Amylothrophic Lateral Sclerosis (ALS), Parkinsons's disease, Ischemia or others, indicates a critical link between cell cycle activation and neuronal cell death. Further evidence in support of this suggestion could be provided by the neuroprotective action of cell cycle blockade under various in vitro paradigms of acute cell death.

In AD, neurodegeneration is not homogenously distributed throughout the brain. It rather shows a distinct pattern with a systematic distribution in space and time. Neuronal re-activation of the cell cycle in AD occurs in those neurons which are potentially vulnerable against cell death, i.e. the pattern of neurons affected by cell cycle re-activation is basically identical to the pattern of neurodegeneration. This indicates that cell cycle re-activation is an early event in the pathogenetic chain eventually leading to cell death. Critical molecular switches of cell cycle activation in neurons, therefore, represent molecular targets suitable for prevention and/or treatment of neurodegenerative disorders. During the experiments resulting in the present invention it was found that progressive neurodegeneration can be prevented through blockade of cell-cycle re-entry of neurons, preferably by use of new gene therapeutic tools allowing for long lasting, safe, neuron-specific and regulated transgene expression.

### Brief description of the drawings

Figure 1: Schematic illustration of the intracellular signalling events triggered by morpho-dysregulation in AD These events involve an aberrant activation of p21ras/MAP-kinase signalling, a loss of differentiation control, the subsequent re-entry and partial completion of the cell cycle and eventually result in cell death.
Figure 2: Neuronal DNA replication in AD assessed by 3 independent methods
   **A**: A prominent 4n-peak is obtained by laser scanning cytometry after PI staining of neurons in AD.
   **B**: Chromogenic in situ hybridisation with a chromosome 17 probe reveals tetraploid neurons (right panel) as well as diploid neurons (left panel) in AD.
   **C**: PCR amplification of alu-repeats after laser capture microdissection of single neurons reveals an additional 4n DNA peak in AD.

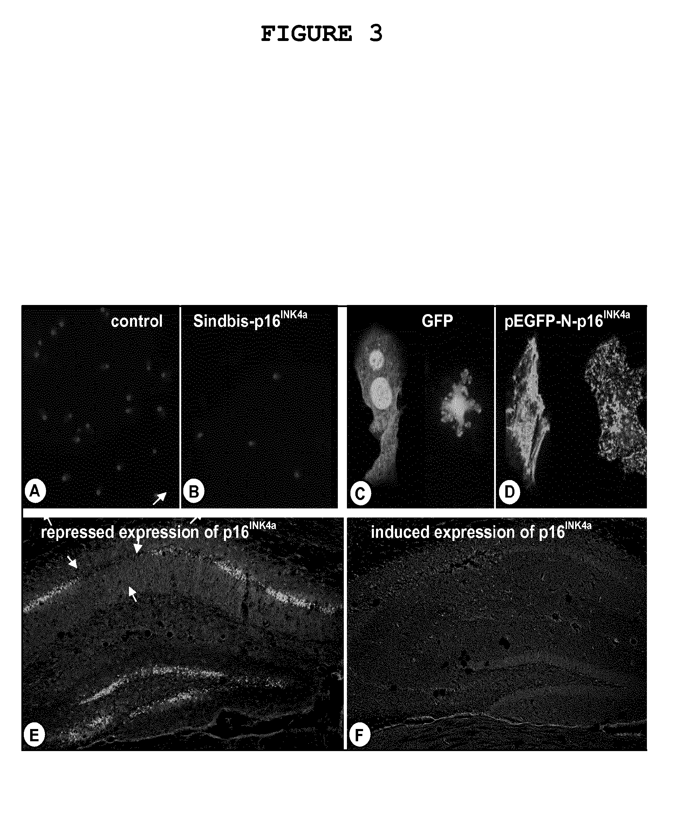

Figure 3: The therapeutic concept of the present invention Protection against degeneration through ectopic expression of p16^{INK4a} in vitro (A-D) and in vivo (E/F)
   **A/B**: microexplants of mice brain (ED 17); **B**: reduced rate of apoptosis induced by okadaic acid (10 nM) after transfection with p16^{INK4a} (TUNEL).
   **C/D (not a subject matter of the invention):** hepatocytes transfected with either GFP or pEGFP-N-p16^{INK4a}. **D**: p16^{INK4a} prevents staurosporine-induced cell death.
   **E/F**: hippocampi of transgenic mice with inducible neuron-specific expression of p16^{INK4a} (CamKII promotor, tet-system). **F**: Induction of p16^{INK4a} expression prevents neuronal death induced by NMDA (Fluorojade staining of degenerating neurons: arrows).

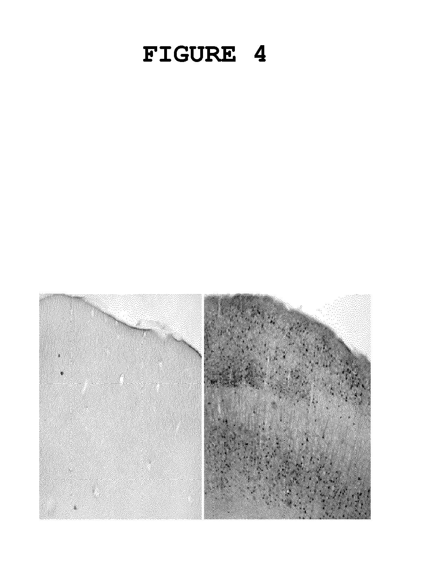

Figure 4: Inducible neuron-specific expression of p16^{INK4a} in the cortex of transgenic mice
   CamKII promoter controlled tTA expression allows regulation of tetO/CMVmin promoter linked p16^{INK4a} expression in dependence of Dox administration (left, plus Dox, off-state; right, without Dox, on-state; immunocytochemical detection of p16^{INK4a})

Thus, the present invention relates to a vector or a mixture of at least two vectors comprising
(a) a nucleic acid molecule encoding (i) a protein of the INK4 family interfering with the biological activity of the cyclin dependent kinase CDK4 and/or CDK6 under the control of an inducible, neuron-specific promoter; and
(b) a (expressible) nucleic acid molecule encoding a transactivator protein that is capable of binding to and activating the inducible promoter of (a) in the presence of an inducer, wherein the nucleic acid molecules (a) and (b) can be present in one vector or, as separate entities, in two vectors.

The protein encoded by the nucleic acid molecule of (a) reduces or inhibits the biological activity of CDK4 and/or CDK6.

Thus, the present invention provides a novel therapeutic concept of preventing progressive neurodegeneration through blockade of cell cycle re-entry of neurons. The invention, inter alia, relates to a gene-therapeutic concept to slow down or even prevent neurodegeneration with high therapeutic efficacy and minimal or no side-effects. The invention uses a gene therapeutic approach that will target the critical molecular regulatory switches CDK4 and CDK6 to slow down or completely shut off the cell cycle in differentiated neurons which will result in rescuing the cell. The concept is based on inhibition of cell-cycle re-entry, a critical trigger of cell death in neurons, and will be accomplished by down-regulation of CDK4 and/or CDK6, e.g., through (i) ectopic expression of its physiological inhibitor p16^{INK4a} or other inhibitors of the INK4 family.

The principle efficacy of the concept the invention is based upon has been proven both under *in vitro* and *in vivo* conditions. A highly efficient protection of neurons through ectopic expression of p16^{INK4a}, a molecular inhibitor of CDK4 and CDK6 in brain slice cultures exposed to strong inductors of neuronal apoptosis such as ocadaic acid could be demonstrated (Figure 3).

Further, in a transgenic mouse model allowing a time dependent regulable and exclusively neuron-specific expression of p16^{INK4a} under control of the CamKII promoter and the tet-expression system, neuroprotective effects against NMDA induced apoptosis and ischemic cell death (Figure 3)

Further, in a transgenic mouse model allowing a time dependent regulable and exclusively neuron-specific expression of p16^{INK4a} under control of the CamKII promoter and the tet-expression system, neuroprotective effects against NMDA induced apoptosis and ischemic cell death (Figure 3)
and reduced apoptosis-related glia reaction could be demonstrated. This strongly indicates that, e.g., down-regulation of CDK4, the major molecular switch of G₀-G₁-transition, by ectopic expression of p16^{INK4a}, restitutes the differentiated neuronal phenotype and rescues neurons as well as other cell types from cell death under a variety of experimental degenerative conditions.

CDK inhibitors that can modulate activity of CDK4 and CDK6 which are critical molecular switches for cell cycle activation belong to the INK4 family (particularly preferred are p16^{INK4a}, p15^{INK4B}, p18^{INK4C} and p19^{INK4D}). Individual INK4 inhibitors have tissue and cell specific properties with respect to inhibition of CDK4/CDK6.

Existing risks with delivery of reagents for gene regulation and specific challenges posed by the central nervous system such as lack of long-lasting gene expression, uncontrolled and unintended transgene expression in non-neuronal cells and uncontrolled intensity of transgene expression can be met by the use of (i) viral and (ii) non-viral vectors for safe gene transfer, (iii) cell type specific recognition systems, (iv) cell-type specific expression system, (v) regulable transgene expression systems and, preferably the combination of one or more of (i) to (v). Widespread but cell-type-specific targeted distribution of expression will be achieved through combination of cell-type specific recognition and expression systems with (vi) convection enhanced delivery (CED).

The modular character of the gene therapeutic tools allows to adapt the above concept in alternative specifications for the treatment of a wide variety of other neurological disorders where unscheduled cell-cycle re-entry of cells is of critical importance such as Parkinsons's disease, stroke, amyotrophic lateral sclerosis or proliferative vitreoretinopathy.

**Table 1**

| **Disorders with involvement of cell cycle regulators that are potential targets for treatment with cell cycle inhibitors** | |
|---|---|
| | **Viral infections** |
| **Cancer** | • human cytomegalovirus (HCMV) |
| **Cardiovascular disease** | • human papillomavirus (HPV) |
| • atherosclerosis | • human immunodeficiency virus (HIV) |
| • cardiac hypertrophy | • herpes simplex virus (HSV) |
| **Nervous system** | |
| • Alzheimer's disease | **Fungal infections** |
| • amylotrophic-lateral sclerosis | **Protozoan disease** |
| • stroke | • malaria |
| | • leishmaniosis |
| **Psoriasis** | • trypanosome |
| **Glomerulonephritis** | |

For controlling expression of the nucleic acid molecule encoding a protein interfering with the biological activity of CDK4 and/or CDK6 different well established on/off regulatory systems are available to regulate gene expression. Basically, they consist of two different expression units: (A) a unit carrying the gene of interest controlled by an inducible promoter (feature (a)), (B) another unit carrying a transactivator protein which is, preferably, constitutively expressed and able to bind a specific substance that mediates activation or repression of inducible promoter activity (feature (b)). For (B), at least the following six systems have been developed for use in human or rodents and that are useful for the present invention: (i) the Tet on/off (GOSSEN AND BUJARD, 1992; GOSSEN ET AL., 1995; BARON AND BUJARD, 2000), (ii) the Pip (pristinamycin-induced protein) on/off (FUSSENEGGER ET AL., 2000); (iii) the macrolide-responsive E.REX technology (WEBER ET AL., 2002), (v) the anti-progestin-dependent (WANG ET AL., 1997), (v) the ecdysone-dependent (CHRISTOPHERSON ET AL., 1992; No ET AL., 1996) and (vi) the rapamycin-dependent system (RIVERA ET AL., 1996; LIBERLES ET AL., 1997).

The Tet-on system is preferred since it is most suitable for applications in patients because:
(i) the inducer doxycycline (Dox) is well tolerated in humans;
(ii) and had been used widely as antibiotic;
(iii) Dox is liposoluble and has considerable tissue penetration properties which inludes the brain (UEBERHAM ET AL., 2005), which is a prerequiste for using the tet-system to develop CNS gene therapies;
(iv) oral administration of Dox allows fast and dose-dependent gene induction/repression switches in *vivo* (AURISICCHIO ET AL., 2001);
(v) The tet-system has already been used for generating viral and non-viral vectors to regulate gene expression; and
(vi) it allows a graded transcriptional response, because the level of gene expression in individual cells correlates directly with the dose of inducer.

In the Tet-on system, the reverse repressor of tetracycline operon (rtetR) is fused to the herpes simplex virus VP16 transcriptional factor to establish the reverse tetracycline-controlled *trans*-activator (rtTA). This binds to an inducible promoter and activates transcription of genes of interest in the presence of tetracycline or its analogous doxycycline. The inducible promoter consists of the Tet operator *tetO* fused to a cytomegalovirus minimal promoter (CMVmin).

A potentially high basic expression in these systems will further be reduced by the following strategies:
- Using tight TRE constructs which are composed of modifed TRE-elements and altered minimal CMV promoter will reduce basal expression of gene of interest.
- The replacement of the CMV promoter by a weak neuron-specific promoter (CamKII or synapsin) fused to the tetO sequence will also reduce basal activity while further increasing neuron specificity. A similar strategy has successfully been used to adapt the albumin promoter (ZABALA ET AL., 2004).
- There are two different chimeric repressors which interact with the TRE-responsible promoter and actively silence the basal expression in the absence of doxycycline: [a] the bacterial tetR protein fused to the repressor domain (KRAB) of the human Kox-1 protein and [b] the tetR protein fused to the human Mad-1 domain, which is involved in the recruitment of mSin3-histone deacetylase complex. For the present invention, the KRAB repressor proteins and a strategy using tetR-Mad-1 are preferred.
- A fourth approach will be cloning the tTS silencer sequence (tetracycline controlled transcriptional silencer, that specifically binds to TRE and further supresses transcription in the absence of Dox) between the CamKII promoter and the transactivator sequence as previously described (SAQR ET AL., 2006).
- Alternatively, varying ratios or delayed application of both the transactivator and the TRE carrying constructs will improve the thightness of the tet system. Alternatively, a single regulable brain-specifc vector carrying both tet elements with an opposite orientation to reduce basal background can be used. If necessary and the expression level is too low the incorporation of the woodchuck posttranscriptional regulatory element (WPRE) at the 3'end will alternatively be considered.
- Incorporating regulatable expression systems into non-integrating episomal based vectors with the promoter and repressor/transactivator elements cloned into separate vectors will allow for tuning expression according to therapeutic requirements and will, thus, further enhance therapeutic safety. It is thus possible to control the relative expression levels by altering the viral ratio and hence avoid problems associated with single vector systems and integration events. Also, non-integrating vectors do not form complex episomal concatamers (seen with AAV vectors that must be used at high titres) that also alter expression profiles.

The person skilled in the art knows various viral vectors that can be used for the present invention. For example, lentiviral vectors are the tools of choice for gene delivery into the central nervous system. They have a relatively large transgene capacity (8-10 kb), can be generated to high titre, have low immunogenicity and unlike retroviral vectors, can efficiently transduce postmitotic neurons to generate stable and long term expression of the transgene (for review see WONG ET AL., 2006). Following entry into target cells, lentiviral vectors stably integrate into the host genome. Safety issues relating to insertional mutagenesis can be avoided by the use of a non-integrating viral vector, for example the adeno-associated vector (AAV). AAV vectors can efficiently transduce neuronal cell types and have low immunogenicity (for review see TENENBAUM ET AL., 2004), however they are limited by transgene capacity (4-5 kb) and have also been shown to integrate into active genes in mice (NAKAI ET AL., 2003). More promisingly, integration-deficient lentiviral vectors, originally described to be inefficient at transducing dividing cells (CASE ET AL., 1999; NALDINI ET AL., 1996), have recently been shown to maintain transgene expression *in vitro* (LU ET AL. 2004; SAENZ ET AL., 2004; VARGAS JR. ET AL., 2004) and *in vivo* (YANEZ-MUNOZ ET AL., 2006; NIGHTINGALE ET AL., 2006). These vectors have been rendered integration-deficient through mutations in the coding sequence of the integrase gene and exist as circular forms in the nucleus without any replication signals. In the adult CNS, efficient and sustained transgene expression from such integration-deficient lentiviral vectors was observed in rodent ocular and brain tissues. Furthermore delivery of a therapeutic gene using these vectors mediated rescue of a rodent model of retinal degeneration (YANEZ-MUNOZ ET AL., 2006). Thus, integration-deficient lentiviral vectors are particularly preferred.

Moreover, the person skilled in the art knows various non-viral vectors that can be used for the present invention. In addition to, and as an alternative to viral vector-mediated delivery, non-viral vector-mediated gene transfer has already successfully been applied to various organs including CNS. Different clinically effective approaches resulting in tumor regression have recently been reviewed (OHLFEST ET AL., 2005B). There might be several advantages of non-viral vectors. They are (i) easy to generate, (ii) simple in their construction and (iii) potentially safer as viral vectors. There is (iv) no risk of uncontrolled replication and (v) their synthesis is less expensive compared to viral vectors (partially because of their easy generation in mammalian cell free systems). Contrary to viral vectors, (vi) there is no pre-existing immunity of human against non-viral vectors that could interfere with transfection efficiency and create potential side-effects (BESSIS ET AL. 2004).

For example, branched and linear polyethylenimines (PEIs) show efficient and versatile gene delivery. PEIs are positively charged and condense negatively-charged DNA to sizes below 200 nm, facilitating cell entry and causing endosomal rupture. The degree of branching affects transfection efficiency (KICHLER 2004). PEIs might be particularly promissing for CNS targeting, possesing several advantages. (i) DNA/PEIs are well tolerated when administered to the CNS (LEMKINE ET AL. 2002; OHLFEST ET AL. 2005_{A}; OH ET AL. 2007), (ii) The brain is an attractive target for PEI where PEIs were found highly enriched even after systemic administration (JOHANSSON ET AL., 2004). To enhance cell specifity (iii) PEIs can be coupled to different ligands possessing high affinity to surface receptors of the target cell.

The vector or mixture of vectors of the present invention further contains a nucleic acid sequence encoding a peptide or polypeptide for neuron-specific targeting. Cell-type specific targeting can be achieved, e.g., by coupling non-viral vectors to peptides and polypeptides, preferably antibodies, against cell-specific surface receptors.

The selection of the appropriate molecular target structure on the cell surface is critical for cell-type specific gene delivery (KIRCHEIS, 2001), and the following aspects need to be considered: Antibodies should have (i) a strong specificity for neurons, they should be (ii) non-toxic and (iii) cause no or only diminished activation of immune cells in vivo. Further, (iv) they should not interfere with critical physiological function. The term "antibody" as used herein describes an immunoglobulin whether natural or partly or wholly synthetically produced. This term also covers any protein having a binding domain which is homologous to an immunoglobulin binding domain. These proteins can be derived from natural sources, or partly or wholly synthetically produced. Examples of antibodies are the immunoglobulin isotypes and the Fab, F(ab¹)₂, scFv, Fv, dAb and Fd fragments.

A suitable target is represented by tyrosine kinase (Trk) A receptors, specifically located on cholinergic neurons which are affected in AD most early and most severly (ARENDT ET AL., 1983). After binding to TrkA receptors, the complete Ab-TrkA-receptor-complex is internalised (LESAUTEUR ET AL., 1996). This allows a proper internalisation of conjugated PEIs as it occurs with the physiological ligand NGF. Alternatively, anti-NGF-antibody can be used. After binding to NGF, the antibody-NGF-complex is also bound to the TrkA receptor followed by accelerated internalisation as compared to NGF alone (SARAGOVI ET AL. 1998). In further embodiments, alternative cell surface molecules of cholinergic neurons, such as p75 neurotrophin receptor (p75NTR), neuronal cell adhesion molecule (NCAM) and nicotinic acetylcholine receptors (nAChR) will specifically be targeted.

In a further embodiment, a modified rabies virus glycoprotein (rvg) recently used to shuttle naked RNAi into neurons (KUMAR ET AL., 2007) will be coupled to PEIs which facilitates stabilisation of transported RNAi. Of note, p75NTR, NCAM and nAChR bind the rabies protein on the cell surface and facilitate internalisation. In this embodiment, endogenic neurotropism of the peptide is used to specifically target cholinergic neurons selectively affected in AD. Further, it allows easy crossing of the blood brain barrier, which in turn allows peripheral administration of this tool.

In order to achieve expression only in the target tissue or organ, e.g., brain, to be treated, the nucleic acid molecules of the vector(s) of the present invention is linked to a tissue specific promoter and used for gene therapy. Thus cell-type specific expression can be achieved by cell-specific control of expression by neuron-specific promoters. Many promoters with preference to neurons have been characterized and were tested in vivo (HIOKI ET AL., 2007) by various shuttle/expression systems. The CamKII and synapsin (SYN) promoters have many advantages, because they are exclusively expressed in neurons (KUGLER ET AL., 2003). Other promoters such as CMV and U1 snRNA mainly mediate gene expression in glial cells. The NSE promoter has only a relative specificity for neurons and is also expressed in glial cells. The SYN promoter shows the highest specifity for neuronal expression (>96%) (HIOKI ET AL., 2007), and has already succesfully been applied for generation of transgenic mice with neuron-specific expression of p21ras (HEUMANN ET AL., 2000; ARENDT ET AL., 2004; GARTNER ET AL., 2005; SEEGER ET AL., 2005; ALPAR ET AL., 2006). Recently, the high neuronal specificity of the CamKII promoter could be demonstrated (UEBERHAM ET AL., 2005; 2006).

Expression level of genes of interest can further be improved by (a) enhancement promoter activity via generating hybrid promoters by fusing with CMV enhancer according to HIOKI ET AL. (2007) or (b) incorporating the wood-chuck hepatitis virus post-transcriptional re-gulatory element (WPRE) at the 3'untranslated region (PATERNA ET AL., 2000).

The present invention also provides vector(s) as described above for use in a method for the prevention or treatment of (a) a neurogenerative disorder.

The present invention also relates to the use of (a) vector(s) as defined above for the preparation of a pharmaceutical composition for the prevention or treatment of (a) a neurogenerative disorder. In a preferred embodiment, said neurogenerative disorder is Alzheimer's disease (AD).

Preferably, the pharmaceutical composition also contains a pharmaceutically acceptable carrier. Examples of suitable pharmaceutical carriers etc. are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the disease, e.g., AD, its localisation and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the disease (e.g. AD), general health and other drugs being administered concurrently.

The delivery of the vectors(s) of the present invention can be achieved, e.g., by direct application to the target site, e.g., the brain or, e.g., by intrathecal, intracerebrospinal, intranasal, intraperintoneal or oral administration.

The blood-brain barrier represents a considerable hurdle to the delivery of therapeutic agents, such as viral vector-mediated gene therapy or PEIs to the brain. The development of techniques to efficiently bypass this barrier would revolutionise the management of neurological diseases. Osmotic disruption of the blood-brain barrier may be achieved by intra-arterial injection of a concentrated mannitol solution prior to drug administration. Although this approach may transiently open-up the endothelial cell tight junctions, drug appears to accumulate in the underlying basement membrane, limiting tissue penetration (MULDOON ET AL., 1999).

In contrast, convection-enhanced delivery (CED) utilises extremely fine intracranial catheters (less than 0.4 mm outer diameter) implanted directly into the brain or spinal cord. Infusion of drugs along these catheters at precisely controlled, low-infusion rates (0.5 to 10 µl/min) leads to drug distribution within the brain extracellular space (KRAUZE ET AL., 2005). Consequently, through accurate positioning of the catheter tip and the use of therapeutic agents that are unable to cross the blood-brain barrier, it is possible to compartmentalise drugs within discrete neuroanatomical structures, limiting the risk of systemic toxicity. This clearly offers tremendous advantages in the delivery of gene therapy.

The principal advantage of CED over other techniques of direct intracranial drug delivery, such as intraparenchymal, intracerebroventricular and intrathecal injection, as well as encapsulated cells and biodegradeable polymers, is that CED does not depend on diffusion to achieve adequate drug distribution. CED distributes therapeutic agents along a pressure gradient generated between the catheter tip and the brain extracellular space. Consequently, in contrast to techniques that are dependent on diffusion, which leads to drug distribution heterogeneously, short distances, down a concentration gradient, CED enables the controlled, homogeneous distribution of drugs over large distances (up to 5 cm from the catheter tip) regardless of their molecular size (GILL ET AL., 2003; GILLIES ET AL., 2005). This clearly offers tremendous advantages in the delivery of vectors used for therapy over clinically significant volumes of brain using a small number of implanted catheters.

The following examples illustrate the invention.

### Example 1

### Study of neuronal DNA replication in AD assessed by 3 different methods

### (A) Slide-based cytometry (SBC)

SBC was performed using a Laser Scanning Cytometer (LSC, CompuCyte Corporation, Cambridge, MA, USA) and the appropriate software WinCyte, version 3.4. The conditions for SBC were optimized for the present application as described previously (Lenz et al., 2004; Mosch et al., 2006). Each fluorescent event was recorded with respect to size, perimeter, x-y position on the object slide and maximum (Max Pixel) and overall integral fluorescence intensity. The entire parahippocampal gyrus was scanned with 80,000-120,000 analyzed cells for each specimen. The relative DNA content of the cells was determined by the integral PI fluorescence values and these data were further analyzed using the cell cycle software ModfitLT, version 2.0 (Verity Software House Inc., Topsham, ME, USA). By this means, cell populations, containing an amount of DNA of 2n, 2n to 4n or 4n could clearly be discriminated (Figure 2A). While most cells were represented by the 2n-peak, an additional 4n-peak (arrow) was clearly obtained for AD brain, which was not present in age-matched healthy control brains.

### (B) Chromogenic in situ hybridization (CISH)

Hybridization was performed with a ZytoDotCEN 17 probe (ZytoVision, Bremerhaven, Germany) which target alpha-satellite-sequences of the centromere of chromosome 17. The digoxigenin labeled probe was immunohistochemically visualized using peroxidase-conjugated Fab fragments of an anti-digoxigenin antibody from sheep (Boehringer-Mannheim, Mannheim, Germany) and nickelammoniumsulfate / DAB / 0.015 % H₂O₂ as chromogen. Fixed human lymphocytes, dropped on object slides and HeLa cells, cultured under standard conditions and grown on cover slips were used as controls. 400-500 neuronal nuclei sampled throughout all cortical layers of the entorhinal cortex were analyzed for each case. Chromogenic in situ hybridization (CISH) with a chromosome 17 probe consistently revealed distinct hybridization spots in all cell types, including neurons, astrocytes and microglial cells. After hybridization of brain sections, two hybridization spots were obtained for the majority of neurons in the entorhinal cortex of both controls and AD patients. In addition, neurons with none, one or three spots were less frequently observed. Figure 2B shows neurons in an advanced case of AD with two spots (left) and four spots (right)(arrows). Scale bar: 10 µm.

### (C) Microdissection and quantitative PCR

Single neurons, indentified by immunoreacitivity for neurofilamants (SMI 311) were cut from brain slices with a laser microdissector (PALM^{®}MicroBeam, P.A.L.M. Microlaser Technologies AG, Bernried, Germany) and subsequently subjected to DNA quantification. DNA content of individual neurons was quantified through real-time PCR amplification of alu repeats (Walker et al., 2003), a class of short interspersed elements in the eukaryotic genome which reach a copy number of about 1 million in primates (Houck et al., 1979; Batzer and Deininger, 2002). Alu repeats were chosen due to their high copy number and low level of polymorphism compared to other short interspersed elements in the eukaryotic genom (Roy-Engel et al., 2001). The residual risk of an artificial influence by different copy numbers or single nucleotide polymorphisms in several individuals was avoided by the intraindividual comparison of two different brain areas of each patient. *Alu* oligonucleotide primers *alu*-for 5'- GTGGCTCACGCCTGTAATCCC -3' and *alu*-rev 5'- ATCTCGGCTCACTGCAACCTC -3', localized in conserved regions of the *alu* repeats, were designed using the 'Primer3' programme (http://frodo.wi.mit.edu/cgi-bin/primer3/primer3 www.cgi). Real-time PCR quantification was accomplished in a Rotor-Gene 2000 (Corbett Research, Sydney, Australia). Data were analyzed by the Rotor-Gene 2000 software Rotorgene, version 4.6, statistics were performed using PlotIT 3.2 (SPE Software, Quebec, Canada). Human lymphocytes treated identically to human brain tissue were used for control. A DNA amount of 2.07 pg ± 0.6 (mean ± SD) and 4.06 pg ± 0.5 was obtained for one single and two lymphocytes, respectively. For each case, at least 20 SMI 311-immunoreactive cells sampled from all layers of the entorhinal cortex were captured with the microdissector and processed for PCR.

Subsequently to CISH, the single cell DNA content was further analyzed in the same cases by laser capture microdissection of neurons in the entorhinal cortex individually identified under the microscope and subsequent PCR amplification of *alu* repeats. The frequency distribution of single cell DNA content obtained by this method is displayed in Figure 2C (upper panel). Comparing AD to controls, a shift towards higher size classes and differences in the shape of the distribution becomes apparent. The distributions of control groups have a single maximum at 2.5-3.5 pg per cell which corresponds to the size for a 2n DNA content as determined in initial validation experiments. In addition, AD groups displayed a second maximum in the size group of 6.5-7.5 pg per cell most likely representing tetraploid neurons (4n) (Figure 2C, lower panel).

### Example 2

### Cloning of p16^{INK4a} and generation of transgenic mice

For cloning of p16^{INK4a} human fibroblast RNA was isolated, reversely transcribed using random pdN6-primers and Superscript II RT (Gibco) and the obtained cDNA was amplified using the following specific primer pairs: p16-forward: 5'-GAG AAC AGA CAA CGG GCG GCG and p16-revers: 5'-CCT GTA GGA CCT TCG GTG ACT.

Following agarose gel electrophoresis the p16^{INK4a} sequence was cloned using sure clone ligation kit (Promega) in a cloning vector (i.e. from PUC18 series [Promega]) resulting in pUC18-p16. This plasmid was transformed in CaCl₂ competent JM109 E.coli cells and cultured on agar plates in the presence of ampicillin. A colony was picked, cultured in LB medium, the plasmid was isolated using Qiagen Maxi-prep and the insert was sequenced. The p16^{INK4a} insert was cut using restriction enzymes and further subcloned into the pSinRep5 vector which was prior linerarized in the multing cloning site with restriction enzymes. The pSinRep5 vector belongs to the Sindbis expression system which was purchased from Invitrogen ("Sindbis Expressions System"; Invitrogen; catalog-Nr. K750-01). The newly generated pSinRep5-p16^{INK4a} vector was linearized and RNA was transcribed using SP6 polymerase. RNA was also transcribed from the DH-BB helper plasmid by SP6 polymerase. DH-BB helper RNA and SinRep5-p16 RNA were mixed with lipofectin (purchased from Gibco) and added to cultured BHK cells. 24 hours after this co-transfection the medium was removed, centrifuged by 2000 x g to remove cell debris and the remaining supernatant containing Sindbis-p16 virus was used as virus stock solution for experiments shown in Example 3.

Starting with the pUC18-p16 plasmid the p16^{INK4a} sequence was cut by restriction enzymes and further subcloned in the expression vector pEGFP-N (Clontech) which was used for experiments shown in Example 3.

For generation of transgenic mice the p16^{INK4a} cDNA was amplified using the following specific primer pairs containing MluI and HindIII sites allowing subcloning into pBI vector (p16-Mlu-F: ctcacgcgtagcgggagcagcatggagccggcg; p16-Hind-R: atcaagcttgctctggttctttcaatcggggat) resulting in pBI-p16^{INK4a}. The transgenic mice with inducible neurospecific-specific expression of p16^{INK4a} were generated using the heterologous tTA system. Briefly, individuals of transgenic line pₜₑₜp16^{INK4a} (C57B1/6-DBA background and generated by microinjection of pBI-p16^{INK4a} in mouse oocytes by conventional methods) carrying the bidirectional transcription unit for luciferase and p16^{INK4a} were interbred with individuals of the transactivator line CamKII (C57B1/6-NMRI background). Animals were housed under a constant day-night cycle of 12:12 hours and fed a standard chow diet (Altromin 1324, Altromin Gesellschaft für Tierernährung, Lage, Germany), with access to water/doxycycline hydrochloride solution ad libitum under all conditions. Animal experiments were carried out in accordance with the European Council Directive of 24 November 1986 (86/609/EEC) and were approved by the local authorities.

Doxycycline hydrochlorid (Sigma, Deisenhofen, Germany, Dox) was dissolved to 50 µg/ml in water and given in brown bottles, which were exchanged twice a week, to prevent transcription. Expression of transgenic proteins was induced by substituting plain water for Dox. P16^{INK4a} expressing mice and controls were used in the experiments described in Example 3.

### Example 3

### Protection against degeneration through ectopic expression of p16^{INK4a} in vitro and in vivo

### (A) Neuroprotection experiments in vitro (results are shown in Figure 3 A+B)

Rat brain slices were transduced with stock dilutions of (A) Sindbis viruses, or (B) Sindbis-p16^{INK4a} viruses. Following treatment of rat brain slices with okadaic acid (10 nM OA, 24 h) which induces neuronal cell death brain slices were incubated with 4% PFA and a TUNEL reaction with dUTP-Rhodamin was performed. Figure 3A: Red colour marks many apoptotic neurons in Sindbis transduced microexplants. Figure 3B shows Sindbis-p16^{INK4a} transduced cultured microexplants (TUNEL; dUTP-Rhodamin) demonstrating reduced neuronal cell death with lower number of apoptotic neurons.

### (B) Neuroprotection experiments in vitro (results are shown in Figure 3 C+D) :

Primary mouse hepatocytes were transfected with pEGFP-N (C) or pEGFP-N-p16 (D) constructs using lipopfectamine method and cultured in 6 well plates in vitro. After 24 hours staurosporine was added to the medium to induce apoptosis. While pEGFP-N transfected hepatocytes died shown by cell blebbing (C), pEGFP-N-p16 transfected survived (D).

### (C) Neuroprotection in vivo (results are shown in Figure 3 E+F)

P16^{INK4a} expressing mice (transgenic p16^{INK4a} is expressed after doxycyclin removal from drinking water) and P16^{INK4a} non-expressing mice (repression of transgenic p16^{INK4a} expression is due to doxycyclin administered in drinking water) were treated with NMDA. By stereotactic apparatus NMDA was administered (2 µg NMDA/µl PBS; injection speed 0.1 µl/ min; injection time 5 min; region: into the hippocampus). After surviving time of 14 days mice were killed, the brains perfused with 4% paraformaledyde and slices were Fluorojade stained for detection of dying neurons. P16^{INK4a} expressing mice (Figure 3F) show low number of apoptotic neurons in contrast to mice with repressed p16^{INK4a} expression (Figure 3E).

Fluoro-Jade B is an anionic fluorochrome which selectively stains both cell bodies and processes of degenerating neurons. The method was slightly adapted from that originally described (Schmued et al., 1997). Sections were mounted onto gelatin-coated (2%) slides, air dried at 50°C for 50 min and immersed in a solution containing 1% sodium hydroxide in 80% ethanol for 3 min. Following incubation for 1 min in 70% ethanol and 2 min washing in distilled water, slides were transferred to a solution of 0.06% potassium permanganate for 15 min on a shaker table. After rinsing in distilled water (1 min), slides were incubated in Fluoro-Jade B staining solution for 20 min. For preparation of staining solution, 10 mg Fluoro-Jade B (Histo- Chem Inc., Jefferson, USA) was dissolved in 100 mL distilled water and 10 mL of this stock solution was diluted with 90 mL of 0.1% acetic acid to give the staining solution. Following staining, slides were rinsed with water, dried and coverslipped. Lesion volumes were determined using series of Fluoro-Jade B-stained slices applying the software NeurolucidaTM (version 5.05.4, MicroBrightField Inc., Williston, USA). Briefly, the lesion was encircled on every tenth Fluoro-Jade B-stained slice and the cross-sectional area was determined by the software NeurolucidaTM.

### Example 4

### Inducible neuron-specific expression of p16^{INK4a} in the cortex (neurons) of transgenic mice (Figure 4, right) after removal of doxycycline compared to repressed transgenic p16^{INK4a} expression (Figure 4, left) in the presence of doxycycline

Transgenic mice with inducible neuron-specific expression of p16^{INK4a} [tTACamKIIa/tTA-responsive promoter (Pₜₑₜ)p16^{INK4a}] were generated using the heterologous tTA system (Baron and Bujard, 2000; Gossen and Bujard, 1992; Gossen et al., 1995). In this tTA system, the transactivator (tTA), a fusion protein of an E. coli-derived tet repressor (tetR) DNA binding domain and the transactivation domain of VP16 protein derived from herpes simplex virus (Gossen and Bujard, 1992) is placed under the control of a CamKIIa promoter, which allows a neuron-specific expression of the tTA protein. The tTA protein can specifically bind to the tet operator (tetO) sequence and subsequently induces the transcription from the adjacent cytomegalovirus (CMV) minimal promoter which is combined with a transgene (p16^{INK4a}). Tetracycline or its derivative Doxycycline (Dox) can prevent binding of tTA to tetO and the transactivation of any transgene cloned behind the CMV promoter is stopped (here p16^{INK4a} expression is prevented). In contrast, removal of Dox allows the induction of transgene expression (here p16^{INK4a}-expression is allowed).

For this purpose a mouse line was used, carrying a chromosomal-integrated pₜₑₜp16^{INK4a} vector (Ueberham et al., 2008), consisting of both the p16^{INK4a} and the luciferase cDNA under control of the bidirectional promoter Pₜₑₜ-bi1 (Baron et al., 1995). For generation of this pₜₑₜp16^{INK4a} line, the human p16^{INK4a}cDNA (see above) was amplified using the following specific primer pairs containing MluI and HindIII restriction endonucleases sites allowing subcloning into pBI-5 vector (CVU89934, GenBank at NCBI, Bethesda, MD, USA; (Baron et al., 1995; Baron and Bujard, 2000) (p16-Mlu-F: ctcacgcgtagcgggagcagcatggagccggcg; p16-Hind-R: atcaagcttgctctggttctttcaatcggggat) resulting in plasmid pBI-p16^{INK4a}. The plasmid pBI-p16^{INK4a} was linerarized by restriction endonucleases and used for generation of transgenic mice by conventional oocyte-injection (C57B1/6-DBA background). The obtained founder mice were tested for transgeneity using standard PCR methods. The pBI vector consists of the bidirectional transcription unit for luciferase and the p16^{INK4a} cDNA which were inherited together, but remain silent in the pₜₑₜp16^{INK4a} mouse line.

To obtain a neuron-specific expression, the Pₜₑₜp16^{INK4a} line was interbred with a mouse line expressing a transactivator protein (tTA) controlled by the calcium-calmodulin kinase IIa promoter (tTACamKIIa- line B; C57B1/6-NMRI background (Mayford et al., 1996)). To prevent uncontrolled expression of p16^{INK4a} during development, doxycycline hydrochloride (Dox; 0.05 mg/mL; Sigma, Taufkirchen, Germany) dissolved in 5% aqueous sucrose solution was supplied in the drinking water while animals were pregnant. Fresh solution was prepared every other day. Non-transgenic siblings obtaining the same dosage of Dox in drinking water showed no toxic effects of the drug. Induction of p16^{INK4a} expression was achieved by omitting Dox from the drinking water, supplying plain water instead. Animals were housed under a constant day-night cycle of 12:12 hours and fed a standard chow diet (Altromin 1324, Altromin Gesellschaft für Tierernährung, Lage, Germany), with access to water or water/doxycycline hydrochloride solution ad libitum under all conditions. Animal experiments were carried out in accordance with the European Council Directive of 24 November 1986 (86/609/EEC) and were approved by the local authorities.

The results are shown in Figure 4: CamKII promoter controlled tTA expression allows regulation of tetO/CMVmin promoter linked p16^{INK4a} expression in dependence of Dox administration (left, plus Dox, off-state; right, without Dox, on-state).

For definition: Plasmid pBI-5 was used to generate pBI-p16^{INK4a} (plasmid) vector; vector pBI-p16^{INK4a} was used to generate Pₜₑₜp16^{INK4a} mouse line.

### List of references

Alpar A, Ueberham U, Bruckner MK, Seeger G, Arendt T, et al. (2006) Different dendrite and dendritic spine alterations in basal and apical arbors in mutant human amyloid precursor protein transgenic mice. Brain Res.1099:189-198.
Arendt T (2000) Alzheimer's disease as a loss of differentiation control in a subset of neurons that retain immature features in the adult brain. Neurobiol.Aging 21:783-796.
Arendt T (2001) Alzheimer's disease as a disorder of mechanisms underlaying structural brain self-organization. Neurosci. 102:723-765.
Arendt T (2003) Synaptic plasticity and cell cycle activation in neurons are alternateve effector pathways The Dr. Jekyll and Mr. Hyde Theory of Alzheimer's disease or The yin and yang of Neuroplasticity. Progr.Neurobiol 71:83-248.
Arendt T, Bigl V, Arendt A, et al. (1983) Loss of neurons in the Nucleus basalis of Meynert in Alzheimer's disease, Paralysis agitans and Korsakoff's disease. Acta Neuropathol 61:101-108.
Arendt T, Brückner M, Bigl V et al. (1995b) Dendritic reorganiza-tion in the basal forebrain under degenerative conditions and its defects in Alzheimer's disease. II. Ageing, Korsakoff's disease, Parkinson's disease, and Alzheimer's disease. J.Comp.Neurol. 351:189-222.
Arendt T, Brückner M, Bigl V et al. (1995c) Dendritic reorgani-zation in the basal forebrain under degenerative conditions and its defects in Alzheimer's disease. III. The basal forebrain compared to other subcortical areas. J.Comp.Neurol. 351:223-246.
Arendt T, Brückner MK, Gertz H-J et al. (1998) Cortical distribution of neurofibrillary tangles in Alzheimer's disease matches the pattern of neurones that retain their capacity of plastic remodelling in the adult brain. Neurosci. 83:991-1002. Arendt T, Gärtner U, Seeger G et al. (2004) Neuronal activation of Ras regulates synaptic connectivity. Eur.J.Neurosc 19: 2953-2966.
Arendt T, Marcova L, Bigl V et al. (1995a) Dendritic reorgani-zation in the basal forebrain under degenerative conditions and its defects in Alzheimer's disease. I.Dendritic organisation of the normal human basal forebrain. J.Comp.Neurol. 351:169-188.
Arendt T, Rödel L, Gärtner U et al. (1996) Expression of the cyclin-dependent kinase inhibitor p16 in Alzheimer's disease. Neuroreport 7:3047-3049.
Arendt T, Schindler C, Brückner MK et al. (1997) Plastic neuronal remodeling is impaired in patients with Alzheimer's disease carrying apolipoprotein ε4 Allele. J.Neurosci. 17: 516-529.
Arendt Th (2008) Differentiation and de-differentiation: Neuronal cell cycle regulation during development and age-related neuro-degenerative disorders. In: Handbook of Neurochemistry (Lajtha A , ed.) Springer pp 157-213.
Aurisicchio L, Bujard H, Hillen W, et al. (2001) Regulated and prolonged expression of mIFN(alpha) in immunocompetent mice mediated by a helper-dependent adenovirus vector. Gene Ther. 8:1817-1825.
Bantounas I, Phylactou LA, Uney JB (2004) RNA interference and the use of small interfering RNA to study gene function in mammalian systems. J.Mol.Endocrinol. 33:545-57.
Baron U, Bujard H (2000) Tet repressor-based system for regulated gene expression in eukaryotic cells: principles and advan-ces. Methods Enzymol. 327:401-421.
Baron,U., Freundlieb,S., Gossen,M., and Bujard,H. (1995). Co-regulation of two gene activities by tetracycline via a bidirectional promoter. Nucleic Acids Res. 23, 3605-3606.
Batzer MA, Deininger PL (2002) Alu repeats and human genomic diversity. Nat Rev Genet 3:370-379.
Bessis N, GarciaCozar FJ, Boissier MC. (2004) Immune responses to gene therapy vectors: influence on vector function and effector mechanisms. Gene Ther. 11 Suppl 1:S10-S17.
Case SS, Price MA, Jordan CT et al. (1999) Stable transduction of quiescent CD34(+)CD38(-) human hematopoietic cells by HIV-1-based lentiviral vectors. PNAS 96:2988-93.
Christoffersen RE, Marr JJ (1995) Ribozymes as human therapeutic agents. J Med Chem. 38:2023-37.
Christopherson KS, Mark MR, Bajaj V et al. (1992) Ecdyste-roid-dependent regulation of genes in mammalian cells by a Drosophila ecdysone receptor and chimeric transactivators. PNAS 89:6314-18.
Emilsson GM, Nakamura S, Roth A et al. (2003) Ribozyme speed limits. RNA 9: 907-18.
Ferri CP, Prince M, Brayne C et al. (2005) Global prevalence of dementia: a Delphi consensus study. Lancet 366: 2112-2117.
Fussenegger M, Morris RP, Fux C et al. (2000) Streptogramin-based gene regulation systems for mammalian cells. Nat. Biotechnol. 18:1203-1208.
Gärtner U, Alpar A, Behrbohm J, Heumann R, Arendt T (2005) Enhanced Ras activity promotes spine formation in synRas mice neocortex. Neuroreport 16:149-152.
Gärtner U, Holzer M, Arendt T (1999) Elevated expression of p21ras is an early event in Alzheimer's disease and precedes neurofibrillary degeneration. Neurosci. 91:1-5.
Gill SS, Patel NK, Hotton GR, et al. (2003) Direct brain infusion of glial cell line-derived neurotrophic factor in Parkinson's disease. Nat Med 9: 589-95.
Gillies GT, Smith JH, Humphrey JA. et al. (2005) Positive pressure infusion of therapeutic agents into brain tissues: mathematical and experimental simulations. Techn.Health.Care13:235-43.
Gonzales-Alegre P., Paulson A.L. (2007) Technology Insights: therapeutic RNA interference - how far from the neurology clinic? Nat. Clin. Pract. Neurology 3 (7): 394-404.
Gossen M, Bujard H (1992) Tight control of gene expression in mammalian cells by tetracycline-responsive promoters. PNAS 89: 5547-5551.
Gossen M, Freundlieb S, Bender G (1995) Transcriptional activa-tion by tetracyclines in mammalian cells. Science 268:1766-1769.
Heumann R, Goemans C, Bartsch D, Lingenhohl K, Waldmeier PC, Hengerer B, Allegrini PR, Schellander K, Wagner EF, Arendt T et al. (2000) Transgenic activation of Ras in neurons promotes hypertrophy and protects from lesion-induced dege-neration. J. Cell Biol. 151:1537-1548.
Highleyman L (1998) FDA approves fomivirsen, famciclovir, and Thalidomide. Food and Drug Administration. BETA. Oct; 5.
Hioki H, Kameda H, Nakamura H et al. (2007) Efficient gene transduction of neurons by lentivirus with enhanced neuron-spe-cific promoters. Gene Ther. 14:872-882.
Houck CM, Rinehart FP, Schmid CW (1979) A ubiquitous family of repeated DNA sequences in the human genome. J Mol Biol 132:289-306.
Johansson A, Nowak G, Moller C et al. (2004) Non-viral delivery of the porphobilinogen deaminase cDNA into a mouse model of acute intermittent porphyria. Mol.Genet. Metab 82:20-26.
Kichler A. (2004) Gene transfer with modified polyethylenimines. J. Gene Med. 6 Suppl 1:S3-10.
Kircheis R, Wightman L, Wagner E. (2001) Design and gene delivery activity of modified polyethylenimines. Adv Drug Deliv Rev. 53: 341-58.
Krauze MT, Saito R, Noble C, et al. (2005) Reflux-free cannula for convection-enhanced high-speed delivery of therapeutic agents. J Neurosurg 103: 923-9.
Kugler S, Kilic E, Bahr M (2003) Human synapsin 1 gene promoter confers highly neuron-specific long-term transgene expression from an adenoviral vector in the adult rat brain depending on the transduced area. Gene Ther. 10:337-347.
Kumar P, Wu H, McBride JL et al. (2007) Transvascular delivery of small interfering RNA to the central nervous system. Nature 448: 39-43.
Kurreck J (2003) Antisense technologies, Improvement through novel chemical modifications. Eur.J.Biochem. 270: 1628-44.
Lemkine GF, Mantero S, Migne C et al. (2002) Preferential trans-fection of adult mouse neural stem cells and their immediate pro-geny in vivo with polyethylenimine. Mol. Cell Neurosci. 19:165-174.
Lenz D, Mosch B, Bocsi J, Arendt T, Tarnok A (2004) Assessment of DNA replication in central nervous system by Laser Scanning Cytometry. Proceedings of SPIE 5322:146-156.
LeSauteur L, Maliartchouk S, Le JH et al. (1996) Potent human p140-TrkA agonists derived from an anti-receptor monoclonal anti-body. J. Neurosci. 16:1308-1316.
Lewin AS, Hauswirth WW (2001) Ribozyme gene therapy: applications for molecular medicine. Trends Mol.Med.; 7: 221-8.
Liberles SD, Diver ST, Austin DJ et al.(1997) Inducible gene ex-pression and protein translocation using nontoxic ligands iden-tified by a mammalian three-hybrid screen. PNAS 94: 7825-30.
Lu R, Nakajima N, Hofmann W et al. (2004) Simian virus 40-based replication of catalytically inactive human immunodeficiency virus type 1 integrase mutants in nonpermissive T cells and monocyte-derived macrophages. J.Virol. 78:658-68.
Mayford,M., Bach,M.E., Huang,Y.Y., Wang,L., Hawkins,R.D., and Kandel,E.R. (1996). Control of memory formation through regulated expression of a CaMKII transgene. Science 274, 1678-1683.
Mosch B, Mittag A, Lenz D, Arendt T, Tarnok A (2006) Laser Scanning Cytometry in human brain slices. Cytometry Part A 69: 135-8.
Mosch B, Morawski M, Mittag A, Lenz D, Tarnok A, Arendt T (2007) Aneuploidy and DNA replication in the mormal human brain and Alzheimer's disease. J.Neurosci. 27:6859-6867.
Muldoon LL, Pagel MA, Kroll RA, et al. (1999) A physiological barrier distal to the anatomic blood-brain barrier in a model of transvascular delivery. AJNR Am J Neuroradiol 20: 217-22.
Nakai H, Montini E, Fuess S et al. (2003) AAV serotype 2 vectors preferentially integrate into active genes in mice. Nat.Genet. 34:297-302.
Naldini L, Blomer U, Gage FH et al. (1996) Efficient transfer, in-tegration, and sustained long-term expression of the transgene in adult rat brains injected with a lentiviral vector. PNAS 93: 11382-8.
Nightingale SJ, Hollis RP, Pepper KA. et al. (2006)Transient gene expression by nonintegrating lentiviral vectors. Mol.Ther.13:1121-32.
No D, Yao TP, Evans RM (1996) Ecdysone-inducible gene expression in mammalian cells and transgenic mice. PNAS 93:3346-51.
Oh S, Pluhar GE, McNeil EA (2007) Efficacy of nonviral gene transfer in the canine brain. J.Neurosurg.107:136-144.
Ohlfest JR, Demorest ZL, Motooka Y et al. (2005a) Combinatorial antiangiogenic gene therapy by nonviral gene transfer using the sleeping beauty transposon causes tumor regression and im-proves survival in mice bearing intracranial human glioblastoma. Mol.Ther. 12:778-788.
Ohlfest JR, Freese AB, Largaespada DA (2005b) Nonviral vectors for cancer gene therapy: prospects for integrating vectors and combination therapies. Curr. Gene Ther. 5:629-641.
Opalinska JB, Gewirtz AM (2002) Nucleic acid therapeutics: a work in progress. Curr Opin Investig Drugs 3:928-33.
Paterna JC, Moccetti T, Mura A et al. (2000) Influence of promoter and WHV post-transcriptional regulatory element on AAV-mediated transgene expression in the rat brain. Gene Ther. 7:1304-1311.
Rivera VM, Clackson T, Natesan S. et al. (1996) A humanized system for pharmacologic control of gene expression. Nat. Med. 2: 1028-1032.
Roy-Engel AM, Carroll ML, Vogel E, Garber RK, Nguyen SV, Salem AH, Batzer MA, Deininger PL (2001) Alu insertion polymorphisms for the study of human genomic diversity. Genetics 159:279-290.
Saenz DT, Loewen N, Peretz M et al. (2004) Unintegrated lentivi-rus DNA persistence and accessibility to expression in nondividing cells: analysis with class I integrase mutants. J.Virol. 78: 2906-20.
Saqr HE, Omran O, Dasgupta S, et al. (2006) Endogenous GD3 ganglioside induces apoptosis in U-1242 MG glioma cells. J Neuro-chem. 96: 1301-14.
Saragovi HU, Zheng W, Maliartchouk S. et al. (1998) A TrkA-selec-tive, fast internalizing nerve growth factor-antibody complex indu-ces trophic but not neuritogenic signals. J. Biol. Chem. 273: 34933-34940.
Seeger G, Gartner U, Arendt T. (2005) Transgenic activation of Ras in neurons increases synapse formation in mouse neocortex. J. Neural Transm. 112:751-761.
Stein CA, Cheng YC (1993) Antisense oligonucleotides as therapeutic agents - is the bullet really magical? Science; 261: 1004-12.
Sullenger BA, Gilboa E (2002) Emerging clinical applications of RNA. Nature 418:252-8.
Tenenbaum L, Chtarto A, Lehtonen E et al. (2004) Recombinant AAV-mediated gene delivery to the central nervous system. J.Gene Med. 6 Suppl 1:S212-S222.
Trülzsch B, Wood M (2004) Application of nucleic acid technology in the CNS. J Neurochem 88:257-65.
Ueberham U, Ueberham E, Bruckner MK, Seeger G, Gartner U, Gruschka H, Gebhardt R, Arendt T. (2005) Inducible neuronal expression of transgenic TGF-beta1 in vivo: dissection of short-term and long-term effects. Eur J Neurosci. 22: 50-64.
Ueberham U, Zobiak B, Ueberham E, Bruckner MK, Boriss H, Arendt T. (2006) Differentially expressed cortical genes contribute to perivascular deposition in transgenic mice with inducible neuron-specific expression of TGF-beta1. Int J Dev Neurosci. 24: 177-86.
Ueberham,E., Lindner,R., Kamprad,M., Hiemann,R., Hilger,N., Woithe,B., Mahn,D., Cross,M., Sack,U., Gebhardt,R., Arendt,T., and Ueberham,U. (2008). Oval cell proliferation in p16(INK4a) expressing mouse liver is triggered by chronic growth stimuli. J. Cell Mol. Med. 12, 622-638.
Van der Flier WM, Scheltens P (2005) Epidemiology and risk factors of dementia. J.Neurol.Neurosurg.Psychiat. 76 supl 5:2-7.
Vargas J, Jr., Gusella GL, Najfeld V. et al. (2004) Novel inte-grase-defective lentiviral episomal vectors for gene transfer. Hum.Gene Ther. 15:361-72.
Walker JA, Kilroy GE, Xing J, Shewale J, Sinha SK, Batzer MA (2003) Human DNA quantitation using Alu element-based polymerase chain reaction. Anal Biochem 315:122-128.
Wang Y, DeMayo FJ, Tsai SY et al. (1997) Ligand-inducible and liver-specific target gene expression in transgenic mice. Nat. Bio-technol. 15:239-243.
Weber W, Fux C, Daoud-el Baba M et al. (2002) Macrolide-based transgene control in mammalian cells and mice. Nat. Biotechnol. 20:901-907.
Wong LF, Goodhead L, Prat C, et al. (2006) Lentivirus-mediated gene transfer to the central nervous system: therapeutic and re-search applications. Hum.Gene Ther. 17:1-9.
Yanez-Munoz RJ, Balaggan KS, MacNeil A et al. (2006) Effective gene therapy with nonintegrating lentiviral vectors. Nat. Med. 12: 348-53.
Zabala M, Wang L, Hernandez-Alcoceba R, et al. (2004) Optimization of the Tet-on system to regulate interleukin 12 expression in the liver for the treatment of hepatic tumors. Cancer Res. 64: 2799-804.
Zhou D, He QS, Wang C. et al. (2006) RNA interference and potential applications. Curr.Top.Med.Chem. 6:901-11.

## Claims

1. A vector or a mixture of at least two vectors comprising
(a) a nucleic acid molecule encoding a protein of the INK4 family, under a cell-specific control of expression by an inducible, neuron-specific promoter; and
(b) a nucleic acid molecule encoding a transactivator protein that is capable of binding to and activating the inducible, neuron-specific promoter of (a)in the presence or absence of an inducer, wherein the nucleic acid molecules (a) and (b) can be present in one vector or, as separate entities, in two vectors.

2. The vector(s) of claim 1, wherein said promoter is the CamKII or synapsin (SYN) promoter.

3. The vector(s) of claim 1 or 2, wherein the transactivator protein is a reverse tetracycline-controlled trans-activator (rtTA).

4. The vector (s) of any one of claims 1 to 3, wherein said vector is coupled to an antibody specific for a cell-specific surface receptor of neurons.

5. The vector(s) of claim 4, wherein said antibody is specific for tyrosine kinase (Trk), nerve growth factor (NGF), p75 neurotrophin receptor (p75NTR), neuronal cell adhesion molecule (NCAM) or nicotinic acetylcholine receptors (nAChR).

6. The vector (s) of any one of claims 1 to 4, wherein said vector is a viral vector.

7. The vector(s) of claim 6, wherein said viral vector is a lentivirus or AAV.

8. The vector (s) of claim 7, wherein said lentivirus is an integration deficient lentivirus.

9. The vector (s) of any one of claims 1 to 5, wherein said vector is a non-viral vector.

10. The vector(s) of any one of claims 1 to 9, wherein said protein of the INK4 family is selected from the group consisting of p19^{INK4D}, p15^{INK4B}, p18^{INK4C} or p16^{INK4a}.

11. The vector(s) as defined in any one of claims 1 to 10 for use in a method for the prevention or treatment of a neurogenerative disorder.

12. The vector(s) for use according to claim 11, wherein said neurogenerative disorder is Alzheimer's disease (AD).

13. The vector for use according to claim 11, wherein said vector is applied to the nervous system through convection enhanced delivery.

14. A pharmaceutical composition comprising the vector(s) of any one of claims 1 to 10.

## Patentansprüche

1. Vektor oder Gemisch aus mindestens zwei Vektoren, umfassend
(a) ein Nukleinsäuremolekül, das für ein Protein der INK4-Familie kodiert, unter einer zellspezifischen Steuerung der Expression durch einen induzierbaren, neuronenspezifischen Promotor; und
(b) ein Nukleinsäuremolekül, das für ein Transaktivatorprotein kodiert, welches in der Lage ist, an den induzierbaren, neuronenspezifischen Promotor von (a) in Gegenwert oder bei Fehlen eines Induktors zu binden und diesen zu aktivieren, wobei die Nukleinsäuremoleküle (a) und (b) in einem Vektor oder, als separate Entitäten, in zwei Vektoren vorliegen können.

2. Vektor/en nach Anspruch 1, wobei der Promotor der CamKII- oder Synapsin(SYN)-Promotor ist.

3. Vektor/en nach Anspruch 1 oder 2, wobei das Transaktivatorprotein ein reverser Tetracyclin gesteuerter Transaktivator (rtTA) ist.

4. Vektor/en nach einem der Ansprüche 1 bis 3, wobei der Vektor an einen Antikörper gekoppelt ist, der für einen zellspezifischen Oberflächenrezeptor von Neuronen spezifisch ist.

5. Vektor/en nach Anspruch 4, wobei der Antikörper für Tyrosinkinase (Trk), den Nervenwachstumsfaktor (NGF), den p75-Neurotrophinrezeptor (p75NTR), das neuronale Zelladhäsionsmolekül (NCAM) oder Nikotinacetylcholinrezeptoren (nAChR) spezifisch ist.

6. Vektor/en nach einem der Ansprüche 1 bis 4, wobei der Vektor ein viraler Vektor ist.

7. Vektor/en nach Anspruch 6, wobei der virale Vektor ein Lentivirus oder AAV ist.

8. Vektor/en nach Anspruch 7, wobei das Lentivirus ein integrationsschwaches Lentivirus ist.

9. Vektor/en nach einem der Ansprüche 1 bis 5, wobei der Vektor ein nicht viraler Vektor ist.

10. Vektor/en nach einem der Ansprüche 1 bis 9, wobei das Protein der INK4-Familie aus der Gruppe bestehend aus p19^{INK4D}, p15^{INK4B}, p18^{INK4C} oder p16^{INK4a} ausgewählt ist.

11. Vektor/en nach einem der Ansprüche 1 bis 10 zur Verwerdung in einem Verfahren zur Verhinderung oder Behandlung einer neurodegenerativen Störung.

12. Vektor/en zur Verwerdung nach Anspruch 11, wobei die neurodegenerative Störung die Alzheimer-Krankheit (AD) ist.

13. Vektor zur Verwerdung nach Anspruch 11, wobei der Vektor durch konvektionsverstärkte Abgabe auf das Nervensystem angewendet wird.

14. Pharmazeutische Zusammensetzung, umfassend den/die Vektor/en nach einem der Ansprüche 1 bis 10.

## Revendications

1. Vecteur ou mélange d'au moins deux vecteurs comprenant
(a) une molécule d'acide nucléique codant une protéine de la famille INK4, sous contrôle d'expression spécifique à la cellule par un promoteur inductible spécifique aux neurones; et
(b) une molécule d'acide nucléique codant une protéine de transactivation qui est à même de se lier à et d'activer le promoteur inductible spécifique aux neurones de (a) en présence ou en l'absence d'un inducteur, où les molécules d'acide nucléique (a) et (b) peuvent être présentes dans un vecteur ou, comme entités séparées, dans deux vecteurs.

2. Vecteur(s) selon la revendication 1, dans le ou lesquels ledit promoteur est le promoteur CaMKII ou synapsin (SYN).

3. Vecteur(s) selon la revendication 1 ou 2, dans le ou lesquels la protéine de transactivation est un transactivateur contrôlé par tétracycline inverse (rtTA).

4. Vecteur(s) selon l'une quelconque des revendications 1 à 3, dans le ou lesquels ledit vecteur est couplé à un anticorps spécifique pour un récepteur de surface spécifique aux cellules de neurones.

5. Vecteur(s) selon la revendication 4, dans le ou lesquels ledit anticorps est spécifique à la tyrosine kinase (Trk), au facteur de croissance du tissu nerveux (NGF), au récepteur de neurotrophine p75 (p75NTR), à la molécule d'adhésion cellulaire neuronale (NCAM) ou aux récepteurs nicotiniques de l'acétylcholine (nACnR).

6. Vecteur(s) selon l'une quelconque des revendications 1 à 4, dans le ou lesquels ledit vecteur est un vecteur viral.

7. Vecteur(s) selon la revendication 6, dans le ou lesquels ledit vecteur viral est un lentivirus ou AAV.

8. Vecteur(s) selon la revendication 7, dans le ou lesquels ledit lentivirus est un lentivirus déficient d'intégration.

9. Vecteur(s) selon l'une quelconque des revendications 1 à 5, dans le ou lesquels ledit vecteur est un vecteur non viral.

10. Vecteur(s) selon l'une quelconque des revendications 1 à 9, dans le ou lesquels ladite protéine de la famille INK4 est choisie parmi le groupe composé de p19^{INK4D}, p15 ^{INK4B}, p18^{INK4C} ou p16^{INK4a}.

11. Vecteur(s) selon l'une quelconque des revendications 1 à 10 destiné(s) à être utilisé(s) dans un procédé pour la prévention ou le traitement d'un trouble neurodégénératif.

12. Vecteur(s) destiné(s) à être utilisé(s) selon la revendication 11, dans le ou lesquels ledit trouble neurodégénératif est la maladie d'Alzheimer (AD).

13. Vecteur destiné à être utilisé selon la revendication 11, dans lequel ledit vecteur est appliqué au système nerveux par délivrance améliorée par convection.

14. Composition pharmaceutique comprenant le ou les vecteurs selon l'une quelconque des revendications 1 à 10.


### SEQUENCE LISTING

<110> Universität Leipzig
<120> Vector(s) containing an inducible gene encoding CDK4/CDK6 inhibitor useful for treating neurodegenerative disorders
<130> U 1069EP
<140> EP 09001521.5
   <141> 2009-02-04
<160> 6
<170> PatentIn version 3.3
<210> 1
   <211> 21
   <212> DNA
   <213> artificial
<220>
   <223> primer alu-for
<400> 1
   gtggctcacg cctgtaatcc c 21
<210> 2
   <211> 21
   <212> DNA
   <213> artificial
<220>
   <223> primer alu-rev
<400> 2
   atctcggctc actgcaacct c 21
<210> 3
   <211> 21
   <212> DNA
   <213> artificial
<220>
   <223> primer p16-forward
<400> 3
   gagaacagac aacgggcggc g 21
<210> 4
   <211> 21
   <212> DNA
   <213> artificial
<220>
   <223> primer p16-reverse
<400> 4
   cctgtaggac cttcggtgac t 21
<210> 5
   <211> 33
   <212> DNA
   <213> artificial
<220>
   <223> primer p16-Mlu-F
<400> 5
   ctcacgcgta gcgggagcag catggagccg gcg 33
<210> 6
   <211> 33
   <212> DNA
   <213> artificial
<220>
   <223> primer p16-Hind-R
<400> 6
   atcaagcttg ctctggttct ttcaatcggg gat 33